# EUROPÄISCHE PATENTSCHRIFT

(11) **EP 2 018 555 B1**
(45) Veröffentlichungstag und Bekanntmachung des Hinweises auf die Patenterteilung: **23.12.2009**
(21) Anmeldenummer: 07725305.2
(22) Anmeldetag: 16.05.2007
(51) Int. Cl.: G01N 33/487, G01N 33/49, G01N 1/34, B01L 3/00, G01N 33/52, G01N 1/18

(54) **SEPARATOR UND SEPARATIONSSTREIFEN**
SEPARATOR AND SEPARATION STRIP
SÉPARATEUR ET BANDES DE SÉPARATION

(30) Priorität: 16.05.2006 DE 202006007817 U
(43) Veröffentlichungstag der Anmeldung: 28.01.2009
(60) Teilanmeldung: 09173187.7
(73) Patentinhaber: Pfaff, Dieter, 86633 Neuburg an der Donau (DE)
(72) Erfinder: Pfaff, Dieter, 86633 Neuburg an der Donau (DE)
(74) Vertreter: Klunker . Schmitt-Nilson . Hirsch
(86) Internationale Anmeldenummer: PCT/EP2007/004390
(87) Internationale Veröffentlichungsnummer: WO 2007/131793

(56) Entgegenhaltungen:
- EP-A- 1 477 804
- EP-B1- 0 278 677
- WO-A-93/03673
- DE-A1- 3 130 749
- DE-U1-202006 007 817

## Beschreibung

Die Erfindung betrifft einen Separator und einen Separationsstreifen zum Separieren von Blutserum oder Blutplasma aus einer Blutprobe.

Aus den japanischen WO 2003/073095, bzw. deren englischer Übersetzung, der EP 1 477 804 A1, ist ein Plasma- oder Serumseparator zum Isolieren von Plasma oder Serum aus Blut bekannt. In dem Ausführungsbeispiel gemäß den Figuren 5 (e) und 6 wird das Serum oder Plasma durch Bewegen eines harten Werkzeugs oder eines Fingernagels aus einer Plasma- oder Serumentnahmeöffnung eines Separationsstreifens herausgedrückt und mit einer Pipette aufgenommen. In dem Ausführungsbeispiel gemäß der Figur 7 erfolgt ein Herausquetschen mittels einer nicht näher beschriebenen Rollenanordnung.

Aufgabe der vorliegenden Erfindung ist es, eine Vorrichtung zur Verfügung zu stellen, mit der eine bestimmte Menge an Blutserum oder Blutplasma auf einfache Weise separiert werden kann, ohne den Separationsteststreifen zu beschädigen oder das Blutserum oder Blutplasma zu verschmutzen.

Diese Aufgabe wird erfindungsgemäß durch einen Separator zur Entnahme von Blutserum oder Blutplasma aus einem Separationsstreifen und durch einen Separationsstreifen zum Abscheiden von Blutserum oder Blutplasma aus einer Blutprobe mittels eines Separators gemäss den unabhängigen Ansprüchen gelöst.

Der erfindungsgemäße Separator zur Entnahme von Blutserum oder Blutplasma einer Blutprobe aus einem Separationsstreifen umfasst ein Oberteil, ein Unterteil und ein Gelenk, welches das Ober- und das Unterteil schwenkbar miteinander verbindet, so dass der Separator auf- und zugeklappt werden kann, wobei in dem zugeklappten Zustand die Unterseite des Oberteils und die Oberseite des Unterteils wenigstens teilweise anliegen. Das Unterteil kann einen Separationsstreifen aufnehmen, der hierfür in eine auf dem Unterteil vorgesehene Vertiefung eingelegt oder eingeschoben werden kann. An dem Unterteil und/oder an der Unterseite des Oberteils ist wenigstens ein Vorsprung ausgebildet der, wenn das Ober- und Unterteil zusammengeklappt sind, lokalen Druck auf den eingelegten Separationsstreifen ausübt. Beim Verschieben oder wenigstens teilweisen Herausziehen des Separationsstreifens wird das Blutserum oder Blutplasma aus dessen hinterem Ende herausgestreift und tritt somit aus dem Separationsstreifen aus.

Gemäss einem Grundgedanken der Erfindung wird durch einen derartigen Separator ein definierter, lokaler Druck auf den Separationsstreifen ausgeübt, und durch Verschieben oder wenigstens teilweises Herausziehen des Separationsstreifens aus dem Separator wird das Blutserum oder Blutplasma auf einfache Weise und berührungs- und beschädigungsfrei aus dem Separationsstreifen entnommen. Der erfindungsgemäße Separator kann somit aufwendige Spezial-Separationsgeräte ersetzen.

In einer ersten Ausführungsform des erfindungsgemäßen Separators ist genau ein Vorsprung in dem Separator vorgesehen, der lokalen Druck auf den Separationsstreifen ausübt. Dieser Vorsprung kann an der Unterseite des Oberteils, an der Oberseite des Unterteils oder an der Vertiefung, insbesondere auf dem Boden der Vertiefung ausgebildet sein.

In einer einfachen Ausführungsform des Separators weist das Unterteil eine Vertiefung auf, in die der Separationsstreifen einschiebbar ist und aus der der Separationsstreifen herausziehbar ist. Durch eine derartige Vertiefung wird die Position des Separationsstreifens im Separator genau definiert, so dass durch einen derartigen Separator eine definierte Menge an Blutserum oder Blutplasma besonders zuverlässig aus dem Separationsstreifen entnommen werden kann.

In einer weiteren Ausführungsform der Erfindung ist ein erster Vorsprung an der Unterseite des Oberteils und auf dem Boden der Vertiefung ein zweiter Vorsprung ausgebildet, die im zusammengeklappten Zustand des Separators zusammenwirken und insbesondere auf den gleichen Bereich des Separationsstreifens von gegenüberliegenden Seiten Druck ausüben. Dadurch wird der lokale Druck besonders gut konzentriert und ein derartiger Separator entnimmt eine definierte Menge an Blutserum oder Blutplasma besonders zuverlässig aus dem Separationsstreifen.

Alternativ zu der Ausführungsform mit zwei gegeneinander wirkenden Vorsprüngen können auch ein Vorsprung und eine gegenüber dem Vorsprung angeordnete Mulde vorgesehen sein, um das Blutserum oder Blutplasma aus dem eingelegten Separationsstreifen herauszustreifen.

Wenn wenigstens ein Vorsprung schwellenförmig ausgebildet ist, lässt sich der Separationsstreifen besonders vorteilhaft und beschädigungsfrei bzgl. des Separators bewegen und verschieben.

Eine besonders gute Entnahmewirkung ergibt sich, wenn wenigstens einer der Vorsprünge quer zur Längsrichtung der Vertiefung ausgebildet ist. Da der Druck hierbei nahezu über die gesamte Breite des Separationsstreifens ausgeübt wird, kann das Blutserum oder Blutplasma besonders effektiv und gleichmässig aus dem Separationsstreifen hinausgestreift werden.

In einer weiteren Ausführungsform hat der wenigstens eine Vorsprung zwei pfeilförmig angeordnete Schenkel. Dabei zeigt die Spitze des Pfeils entweder in die Richtung, in welcher der Separationsstreifen aus dem Separator gezogen wird, oder zu dem Gelenk hin. Dadurch wird das Blutserum oder Blutplasma beim Herausziehen des Separationsstreifens in der Mitte des Separationsstreifens konzentriert und mittig aus dem Separationsstreifen hinausgestreift.

In einer weiteren Ausführungsform ist der wenigstens eine Vorsprung bogenförmig ausgebildet, wobei die Öffnung des Bogens gegen die Richtung zeigt, in die der Separationsstreifen aus dem Separator gezogen wird. Dadurch wird das Blutserum oder Blutplasma beim Herausziehen des Separationsstreifens in der Mitte des Separationsstreifens konzentriert und mittig aus dem Separationsstreifen hinausgestreift.

In einer alternativen Ausführungsform ist der wenigstens eine Vorsprung als Rolle oder Walze ausgebildet. Dabei kann das Blutserum oder Blutplasma beschädigungsfrei aus dem Separationsstreifen hinausgestreift werden. Dadurch lässt sich der Separationsstreifen besonders leicht aus dem Separator ziehen und es wird vermieden, dass der Separationsstreifen durch zu kraftvolles Ziehen zerrissen wird. Die Rolle oder Walze ist dabei insbesondere um eine in das Oberteil bzw. Unterteil eingelassene Achse rotierbar.

Gemäß einer weiteren Ausführungsform der Erfindung ist wenigstens ein Antriebsmotor für die Rolle oder Walze vorgesehen, mittels dessen der eingelegte Separationsstreifen automatisch bewegt und verschoben werden kann.

Gemäss einer weiteren Ausführungsform der Erfindung hat das Oberteil nahe dem wenigstens einen Vorsprung eine Aussparung, so dass das von dem Separationsstreifen entnommene Blutserum oder Blutplasma auf dem Unterteil des Separators aufliegt und dass durch diese Aussparung auf das Blutserum oder Blutplasma im zusammengeklappten Zustand des Separators zugegriffen werden kann. Dadurch wird die Benutzerfreundlichkeit des Separators weiter erhöht, zumal das aus dem Separationsstreifen hinausgestreifte Blutserum oder Blutplasma besonders einfach bspw. mittels einer Pipette oder Küvette entnommen werden kann, ohne dass der Separator zuvor aufgeklappt zu werden braucht.

In einer weiteren Ausführungsform der Erfindung ist der wenigstens eine Vorsprung aus einem elastischen Material, insbesondere aus Gummi ausgebildet. Alternativ dazu kann der wenigstens eine Vorsprung auch als Metallzunge ausgebildet sein, die durch ihre Konstruktion elastisch ist. Dadurch wird der Separationsstreifen einerseits mit einer hinreichend grossen Kraft zusammengedrückt, um das Blutserum oder Blutplasma zuverlässig aus dem Separationsstreifen hinauszustreichen, andererseits verhindert die Elastizität des wenigstens einen Vorsprungs, dass der Separationsstreifen im Betrieb beschädigt wird, bspw. wenn der Separationsstreifen schnell aus dem Separator gezogen wird oder wenn das Ober- und Unterteil übermässig stark zusammengedrückt werden.

Das Ober- und Unterteil können aus einem widerstandsfähigem, schlagzähem und leicht desinfizierbarem Material, wie aus einem geeigneten Kunststoff oder aus Metall hergestellt sein.

In einer weiteren Ausführungsform der Erfindung ist das Gelenk kostengünstig durch Bohrungen in dem Ober- und Unterteil ausgebildet, durch die ein Gelenkstift geführt wird. Der Gelenkstift kann ebenfalls aus einem geeigneten Material wie Metall oder Kunststoff hergestellt sein. Der Gelenkstift kann an mindestens einem Kopfende eine schlitz- oder kreuzförmige Vertiefung aufweisen, um den Gelenkstift besonders einfach in die Bohrung einzuführen und um ihn mittels eines Schraubendrehers in die Bohrungen einzuführen oder zu einzudrehen.

In einer weiteren Ausführungsform sind Permanentmagnete in dem Oberteil sowie in dem Unterteil ausgebildet, welche so angeordnet sind, dass sie im zusammengeklappten Zustand einander gegenüber liegen und das Oberteil und das Unterteil durch die magnetische Anziehungskraft zusammenhalten. Dadurch werden das Oberteil und das Unterteil mit einer genau definierten Kraft zusammengehalten, so dass eine definierte Kraft auf den Separationsstreifen ausgeübt wird, welche hinreichend gross ist, um das Blutserum oder Blutplasma aus dem Separationsstreifen hinauszudrücken, ohne den Separationsstreifen zu beschädigen.

In einer alternativen Ausführungsform ist entweder nur das Oberteil oder nur das Unterteil mit Permanentmagneten ausgestattet. Das andere Teil, welches nicht mit Permanentmagneten ausgestattet ist, ist aus Metall oder mit magnetischen Metallplättchen versehen. Die Permanentmagnete und die Metallplättchen sind so angeordnet, dass sie im zusammengeklappten Zustand einander gegenüber liegen und das Oberteil und das Unterteil durch die magnetische Kraft zusammengehalten werden. Diese Ausführungsform ist kostengünstig, da weniger Permanentmagnete benötigt werden.

Nicht Teil der Erfindung ist ein Separator zur Entnahme von Blutserum oder Blutplasma einer Blutprobe aus einem Separationsstreifen, der ein Unterteil und ein gegenüber dem Unterteil verschiebbares Oberteil aufweist. Das Unterteil kann einen Separationsstreifen aufnehmen, der hierfür auf das Unterteil oder in eine auf dem Unterteil vorgesehene Vertiefung eingelegt oder eingeschoben werden kann. An der Unterseite des Oberteils ist wenigstens ein Vorsprung vorgesehen, der lokalen Druck auf den eingelegten Separationsstreifen ausübt. Beim Verschieben des Oberteils in Richtung des vorderen Endes des eingelegten Separationsstreifens wird das Blutserum oder Blutplasma aus dem vorderen Ende herausgestreift und tritt somit aus dem Separationsstreifen aus.

Durch einen derartigen Separator wird ein definierter, lokaler Druck auf den Separationsstreifen ausgeübt, durch Verschieben des Oberteils gegenüber dem Unterteil wirkt der Vorsprung auf den Separationsstreifen und somit wird das Blutserum oder Blutplasma berührungs- und beschädigungsfrei aus dem Separationsstreifen entnommen.

Durch das Anordnen und Verschieben des Oberteils in einem definierten Abstand zum Unterteil wird durch den Vorsprung ein konstanter Druck auf den Separationsstreifen ausgeübt und das Blutserum oder Blutplasma mit gleichbleibender Effizienz aus dem Separationsstreifen herausgestreift.

In einer ersten Ausführungsform dieses nicht erfindungsgemäßen Separators ist der Vorsprung eine Rolle oder eine Walze, wodurch eine schonende Behandlung des Separationsstreifens erreicht, eine Beschädigung des Separationsstreifens vermieden und ein besonders effizientes Herausstreifen des Blutserums oder Blutplasmas gewährleistet wird. Die Rolle oder Walze ist insbesondere um eine in das Oberteil eingelassene Achse rotierbar.

Gemäß einer weiteren Ausführungsform, die nicht teil der Erfindung ist, kann ein elektrischer Antriebsmotor für die Rolle vorgesehen sein, der beispielsweise im Oberteil integriert ist, um das Blutserum oder Blutplasma automatisch und mit einer vorgegebenen Geschwindigkeit und somit noch zuverlässiger aus dem Separationsstreifen herauszustreifen.

Der Verschiebemechanismus des Oberteils gegenüber dem Unterteil kann beliebig ausgeführt werden. Gemäß einer Ausführungsform, die nicht teil der Erfindung ist, sind Führungsschienen vorgesehen, auf denen das Oberteil gegenüber dem Unterteil verschoben werden kann, beispielsweise mittels in die Führungsschienen eingreifende und in diesen laufende Gleiter.

Um eine maximale Verschiebeposition des Oberteils gegenüber dem Unterteil zu gewährleisten, kann wenigstens ein Anschlag vorgesehen sein. Dieser kann beispielsweise durch das Ende der Führungsschiene gebildet sein, gegen die ein Gleiter oder Gleitelement anschlägt.

Gemäß einer weiteren Ausführungsform, die nicht teil der Erfindung ist, hat das Oberteil eine Aussparung, die so angeordnet ist, dass das aus dem hinteren Ende des Separationsstreifens ausgetretene Blutserum oder Blutplasma zugänglich ist, wenn das Oberteil gegenüber dem Unterteil verschoben worden ist und durch Einwirkung des Vorsprungs das Blutserum oder das Blutplasma aus dem hinteren Ende des Separationsstreifens herausgetreten ist. Diese Aussparung kann insbesondere in einem mittleren Bereich des hinteren Endes des Oberteils angeordnet sein.

Alternativ zu einer Aussparung kann das Oberteil kürzer ausgeführt sein, so dass dessen Ende in der maximalen Verschiebeposition so angeordnet ist, dass das aus dem hinteren Ende des Separationsstreifens ausgetretene Blutserum oder Blutplasma zugänglich ist.

Gemäß einer weiteren Ausführungsform, die nicht Teil der Erfindung ist weist die Vertiefung an wenigstens einer Längsseite, wenigstens eine Ausformung auf. Insbesondere kann an beiden Längsseiten jeweils eine Ausformung vorgesehen sein.

Bei der erfindungsgemäßen Schwenkvariante des Separators bildet die Ausformung mit einem vorderen und einem hinteren Anschlag einer Verengung des Separationsstreifens einen Anschlag, so dass der Separationsstreifen über die durch die Anschläge vorgegebene Distanz bewegt werden kann und durch den wenigstens einen Vorsprung eine definierte Menge an Blutserum oder Blutplasma aus dem Separationsstreifen herausgestreift wird. Dadurch wird die Benutzerfreundlichkeit weiter erhöht, zumal der Separationsstreifen nicht vollständig aus dem Separator gezogen muss, sondern nur über eine durch diese Anschläge definierte Distanz bewegt zu werden braucht.

Bei der nicht Schiebevariante des Separators ist diese Ausformung vorzugsweise so bemessen, dass sie in einen damit korrespondierenden Ausschnitt des Separationsstreifens eingreift und somit den Separationsstreifen in oder auf dem Unterteil fixiert.

In einer weiteren Ausführungsform ist eine Zeitmesseinheit mit einem Signalgeber in dem Oberteil oder dem Unterteil vorgesehen. Die Zeitmesseinheit misst ein vorgebbares Zeitintervall, und der Signalgeber gibt nach Ablauf dieses Zeitintervalls ein optisches oder akustisches Signal aus. Die Zeitmesseinheit kann über Bedientasten verfügen, mittels derer das erforderliche Zeitintervall manuell eingestellt werden kann. Der Beginn der Zeitintervallmessung kann der Zeitmesseinheit durch Betätigen einer Bedientaste, durch Einlegen des Separationsstreifenswas durch einen Schalter oder Sensor erfasst werden kann, bei der Schwenkvariante des Separators durch Zusammenklappen von Ober- und Unterteil, was durch einen Schalter oder Sensor erfasst werden kann, und bei der Schiebevariante des Separators durch Aufsetzen des Oberteils auf das Unterteil, was durch einen Schalter oder Sensor erfasst werden kann, angezeigt werden. Der Signalgeber kann in Form einer Digitalanzeige ausgebildet sein, deren Ziffern nach Ablauf des Zeitintervalls zu leuchten oder zu blinken beginnen. Der Signalgeber kann auch eine LED oder ein Summer sein.

Die Erfindung betrifft auch einen Separationsstreifen zum Abscheiden von Blutserum oder Blutplasma aus einer Blutprobe mittels eines Separators. Ein derartiger Separationsstreifen wird auch als Getter bezeichnet, und er umfasst einen Blutseparationsteil, einen Halteteil zum Abdecken und Festhalten des Blutseparationsteils, einen Bluteinführungsabschnitt, der in einem das vordere Ende des Blutseparationsteils abdeckenden Abschnitt des Halteteils geformt ist und eine Blutentnahmeöffnung, die in dem das hintere Ende des Blutseparationsteils abdeckenden Abschnitt des Halteteils geformt ist. Der Separationsstreifen weist an mindestens einer Längsseite wenigstens eine Verengung mit einem vorderen und einem hinteren Anschlag auf, so dass der Separationsstreifen über die durch die Anschläge vorgegebene Distanz bewegt werden kann. Dadurch wird erreicht, dass der Separationsstreifen in Verbindung mit einer an der Längsseite der Vertiefung oder einer Führung eines Separators ausgebildeten Ausformung über eine durch den Abstand des vorderen und hinteren Anschlags und durch die Breite der Ausformung an der Längsseite der Vertiefung des Separators definierte Länge bewegt werden kann. Es ist demzufolge nicht erforderlich, den Separationsstreifen ganz aus dem Separator herauszuziehen oder den Separator aufzuklappen, um das entnommene Blutserum oder Blutplasma weiter verarbeiten zu können.

In einer vorteilhaften Weiterbildung des Separationsstreifens sind die Anschläge der Verengung des Separationsstreifens dafür bestimmt, gegen eine Ausformung im Unterteil, insbesondere gegen eine Ausformung am Seitenrand der Vertiefung des Separators anzuschlagen.

In einer besonders vorteilhaften Ausführungsform ist der Separationsstreifen so ausgebildet, dass die Blutprobe durch einen Blutein Führungsabschnitt in den Blutseparationsteil eingeführt werden kann, dass die eingeführte Blutprobe durch Kapillarwirkung derart trennbar ist, dass sich das Blutserum oder Blutplasma im vorderen Ende des Blutseparationsteils und die roten Blutkörperchen im hinteren Ende des Blutseparationsteils ansammeln. Dadurch wird gewährleistet, dass ausschliesslich das Blutserum oder Blutplasma, nicht aber die roten Blutkörperchen aus dem Separationsstreifen herausgestreift und zur Weiterverarbeitung separiert werden.

Eine besonders einfache und zuverlässig arbeitende Ausführungsform des Separationsstreifens umfasst eine rechteckig ausgebildete Verengung an wenigstens einer Längsseite des Separationsstreifens. Eine derartige rechteckige Verengung ist besonders einfach durch ein geeignetes Stanzwerkzeug herzustellen und der Abstand zwischen dem vorderen und hinteren Anschlag der Verengung ist genau definiert.

Wenn der Separationsstreifen eine halbkreisförmige oder V-förmige Seite aufweist, dann lässt sich das Blutserum oder Blutplasma besonders gut in der Mitte des Separationsstreifens sammeln und mittig aus diesem herausdrücken.

In einer besonders vorteilhaften Ausführungsform wird ein Separator, dessen Vertiefung an mindestens einer Längsseite mindestens eine Ausformung aufweist, und ein Separationsstreifen, der an einer Längsseite mindestens eine Verengung aufweist, verwendet.

Bei dem erfindungsgemäßen Separator der Schwenkvariante wird der Separationsstreifen an seinem vorderen Ende so weit aus dem Separator gezogen, bis die mindestens eine Ausformung an der Seitenwand der im Unterteil des Separators ausgebildeten Vertiefung an das vordere Ende des Ausschnitts des Separationsstreifens anschlägt. Dadurch wird der Separationsstreifen über eine zuvor genau definierte Länge bewegt, so dass eine definierte Menge an Blutserum oder Blutplasma aus dem Separationsstreifen hinaus gedrückt wird.

In einer besonders vorteilhaften Ausführungsform wird das ausgetretene Blutserum oder Blutplasma mit einer Küvette aufgenommen, so dass es unmittelbar für die weitere Verarbeitung bzw. Analyse zur Verfügung steht.

Die Erfindung wird nachfolgend anhand von Ausführungsbeispielen mit Bezug auf die beiliegenden Figuren ausführlich beschrieben.

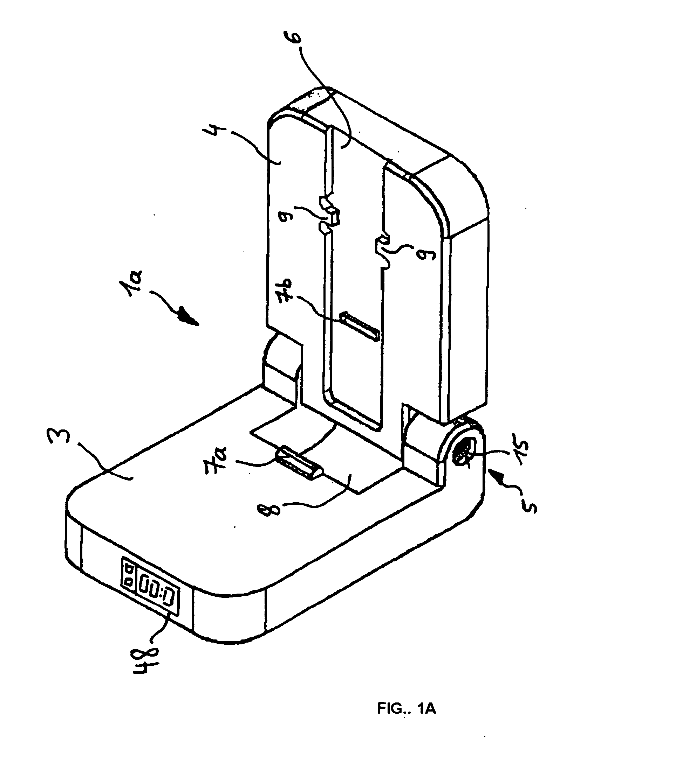

- Fig. 1a: zeigt eine perspektivische Ansicht eines erfindungsgemäßen ersten Separators gemäss einem Ausführungsbeispiel im aufgeklappten Zustand;

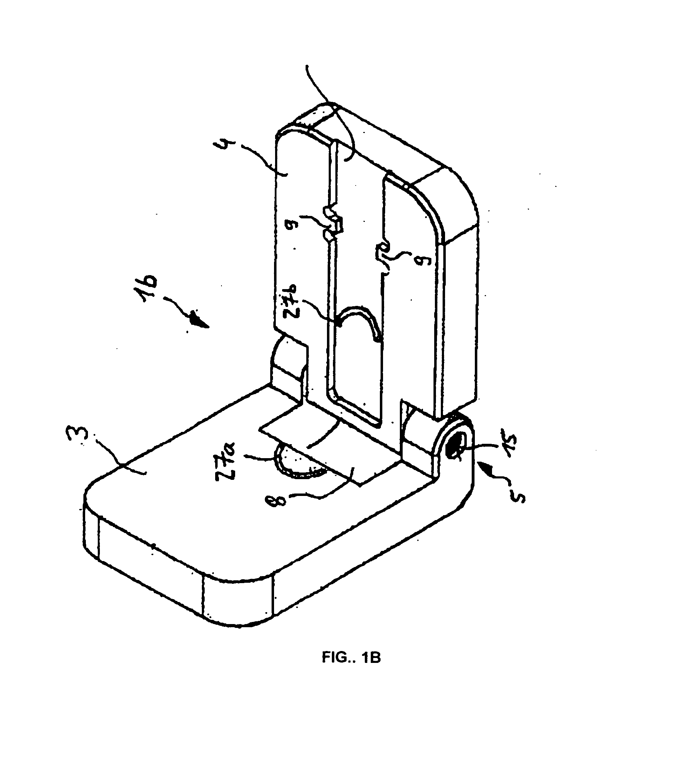

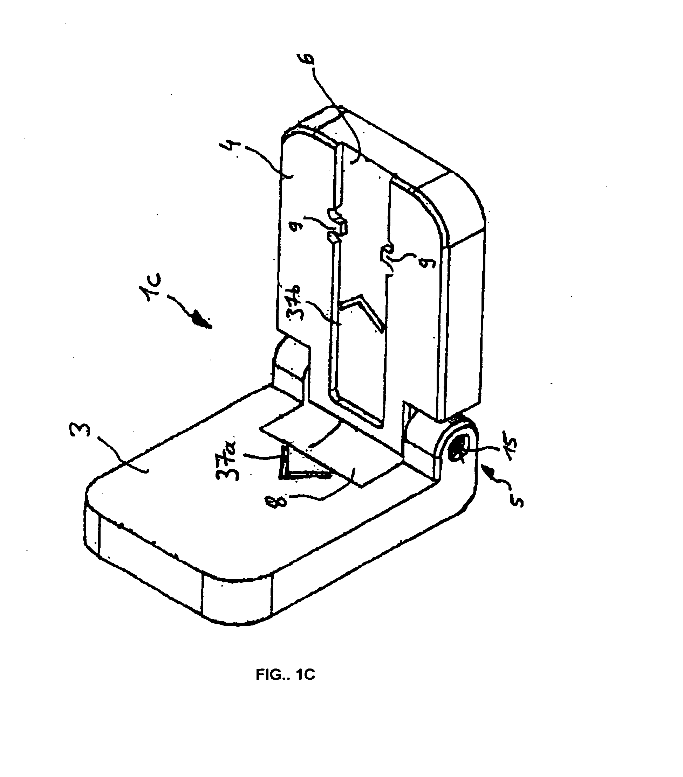

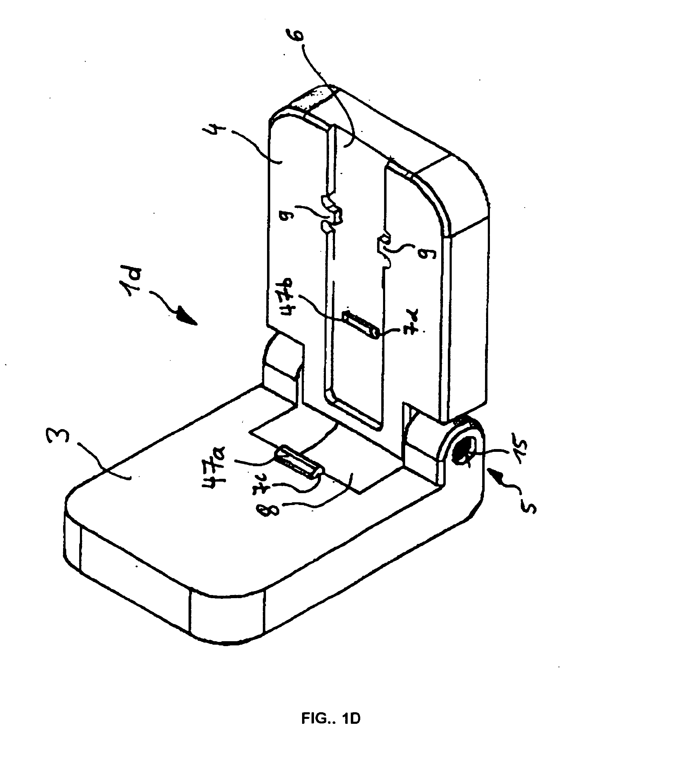

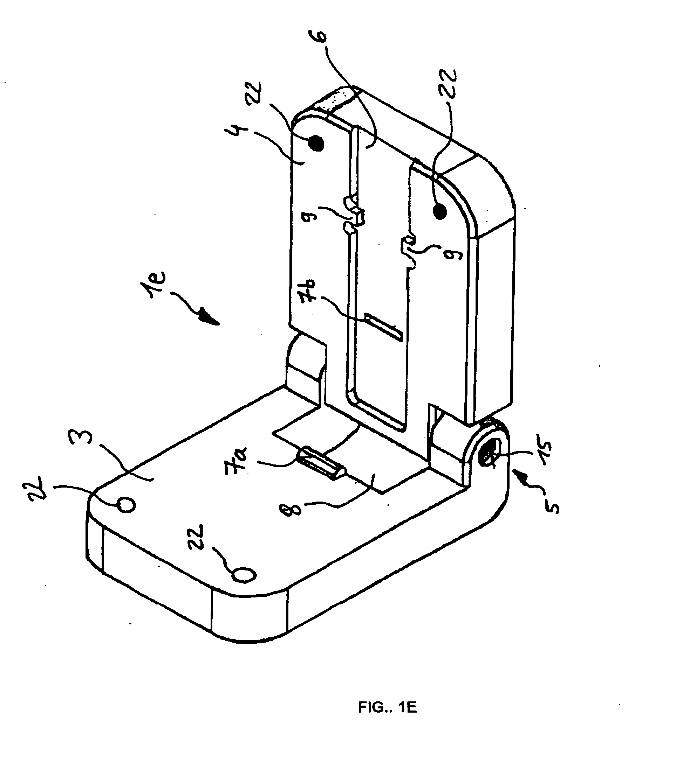

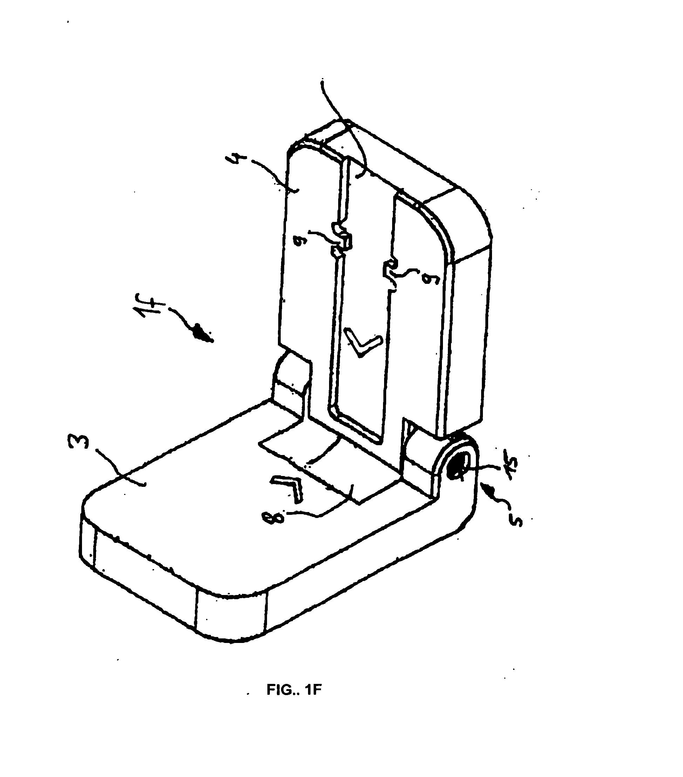

- Fig. 1 b: zeigt eine perspektivische Ansicht eines erfindungsgemäßen zweiten Separators gemäss einem Ausführungsbeispiel im aufgeklappten Zustand;
- Fig. 1c: zeigt eine perspektivische Ansicht eines erfindungsgemäßen dritten Separators gemäss einem Ausführungsbeispiel im aufgeklappten Zustand;
- Fig. 1d: zeigt eine perspektivische Ansicht eines erfindungsgemäßen vierten Separators gemäss einem Ausführungsbeispiel im aufgeklappten Zustand;
- Fig. 1 e: zeigt eine perspektivische Ansicht eines erfindungsgemäßen fünften Separators gemäss einem Ausführungsbeispiel im aufgeklappten Zustand;
- Fig. 1f: zeigt eine perspektivische Ansicht eines erfindungsgemäßen sechsten Separators gemäss einem Ausführungsbeispiel im aufgeklappten Zustand;

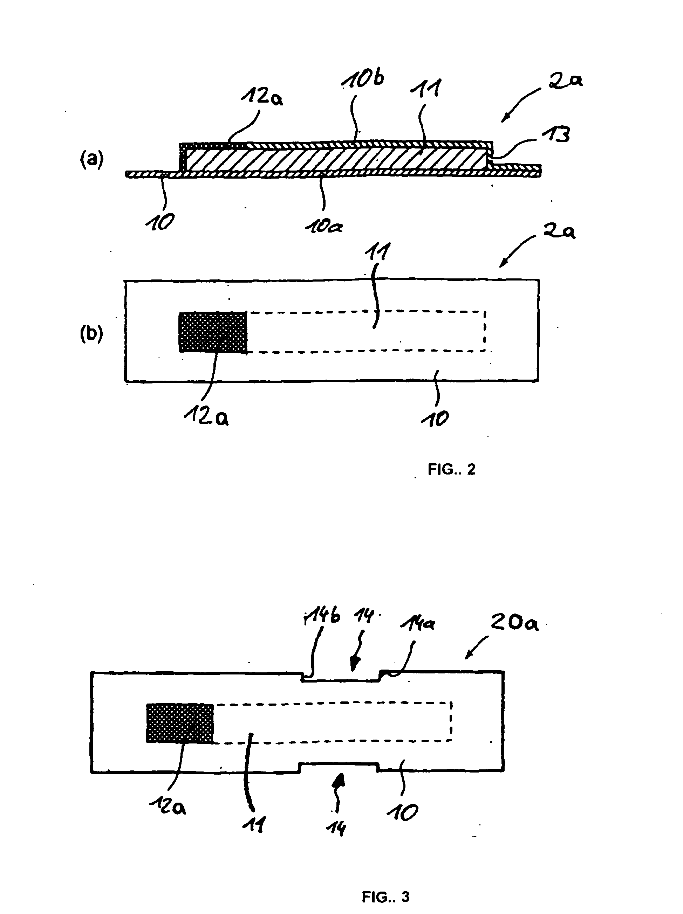

- Fig. 2a: zeigt eine Schnittdarstellung eines ersten, nicht erfindungsgemäßen, Separationsstreifens gemäss einem Ausführungsbeispiel;
- Fig. 2b: zeigt eine Draufsicht auf den ersten Separationsstreifen aus Fig. 2a;
- Fig. 3: zeigt eine Draufsicht auf einen zweiten, erfindungsgemäßen, Separationsstreifen gemäss einem Ausführungsbeispiel;

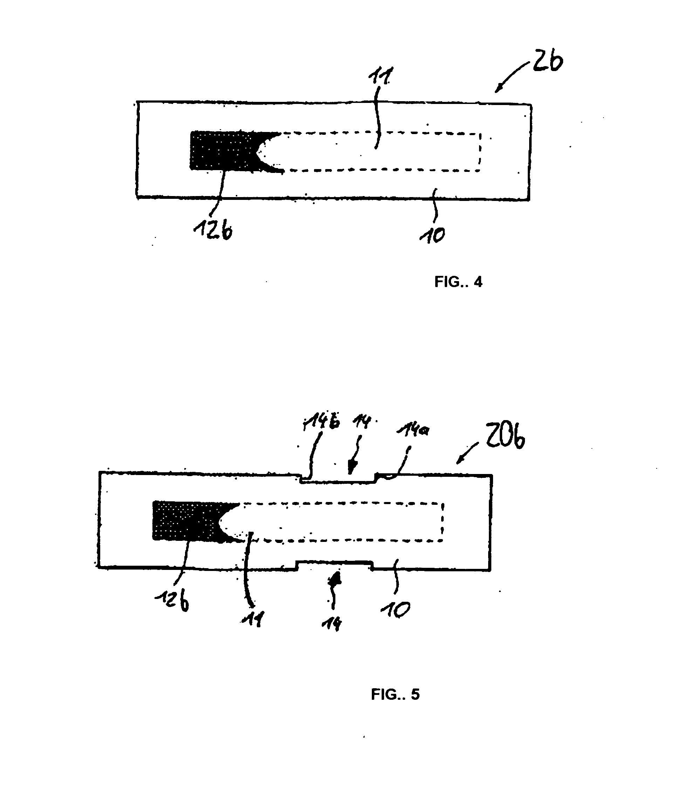

- Fig. 4: zeigt eine Draufsicht auf einen dritten, nicht erfindungsgemäßen, Separationsstreifen gemäss einem Ausführungsbeispiel;
- Fig. 5: zeigt eine Draufsicht auf einen vierten, erfindungsgemäßen, Separationsstreifen gemäss einem Ausführungsbeispiel;

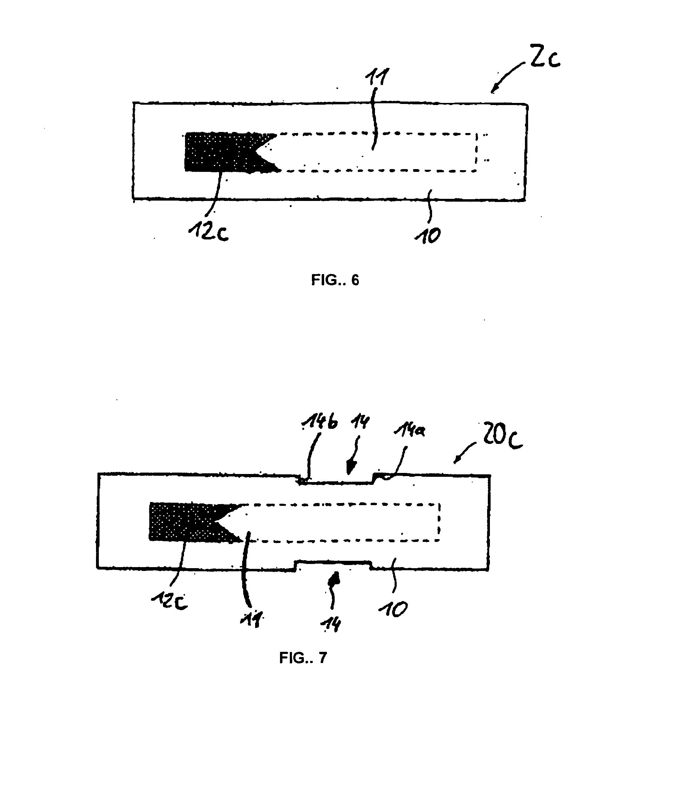

- Fig. 6: zeigt eine Draufsicht auf einen fünften, nicht erfindungsgemäßen Separationsstreifen gemäss einem Ausführungsbeispiel;
- Fig. 7: zeigt eine Draufsicht auf einen sechsten, erfindungsgemäßen, Separationsstreifen gemäss einem Ausführungsbeispiel;

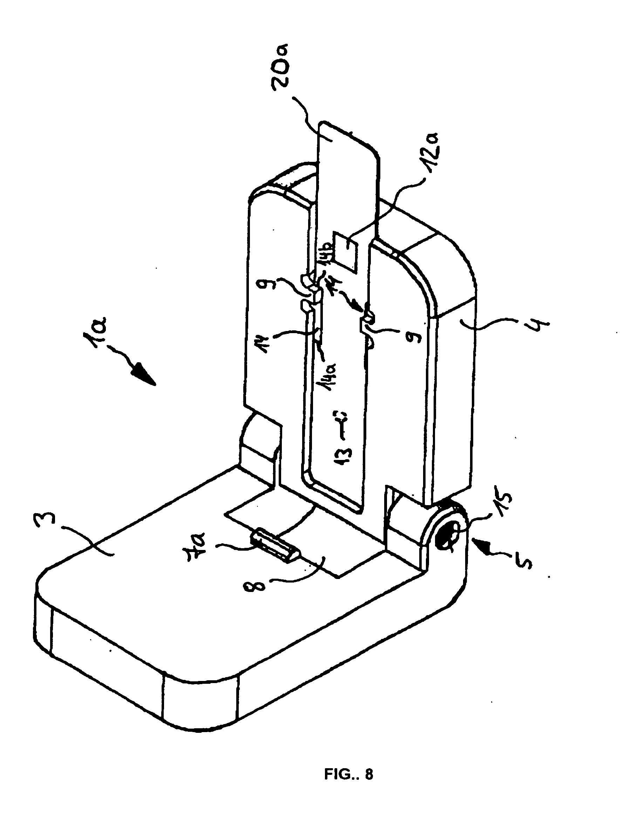

- Fig. 8: zeigt eine perspektivische Ansicht des ersten Separators aus Fig. 1a im aufgeklappten Zustand, in den der Separationsstreifen aus Fig. 3 eingelegt ist;

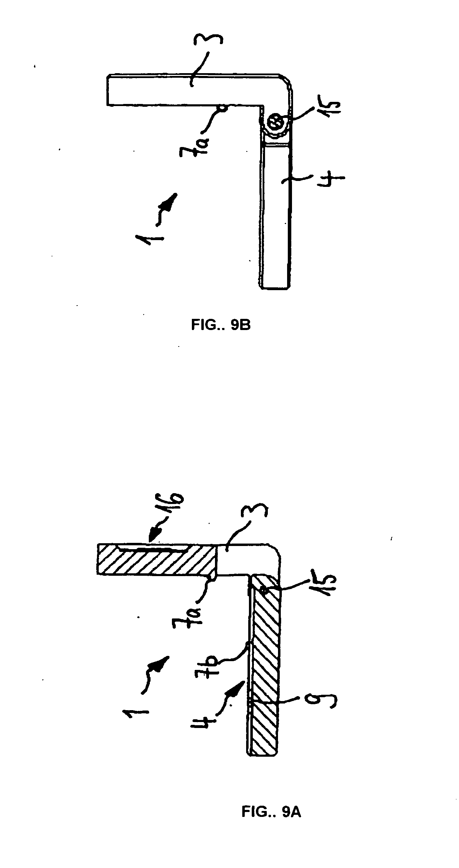

- Fig. 9a: zeigt eine Seitenansicht des ersten Separators aus Fig. 1a im aufgeklappten Zustand;
- Fig. 9b: zeigt eine Schnittdarstellung des ersten Separators aus Fig. 1a im aufgeklappten Zustand;

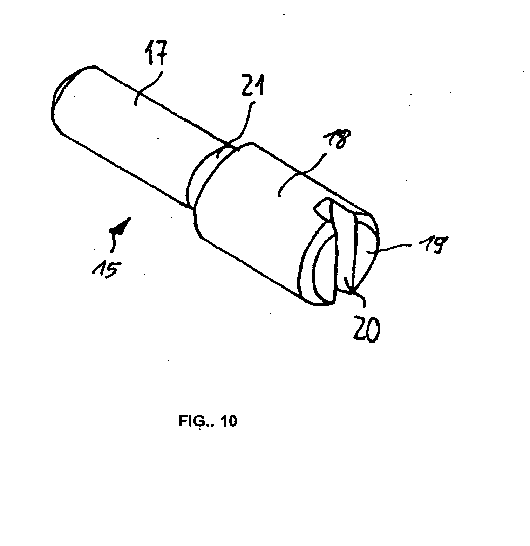

- Fig. 10: zeigt eine perspektivische Ansicht eines Gelenkstiftes;

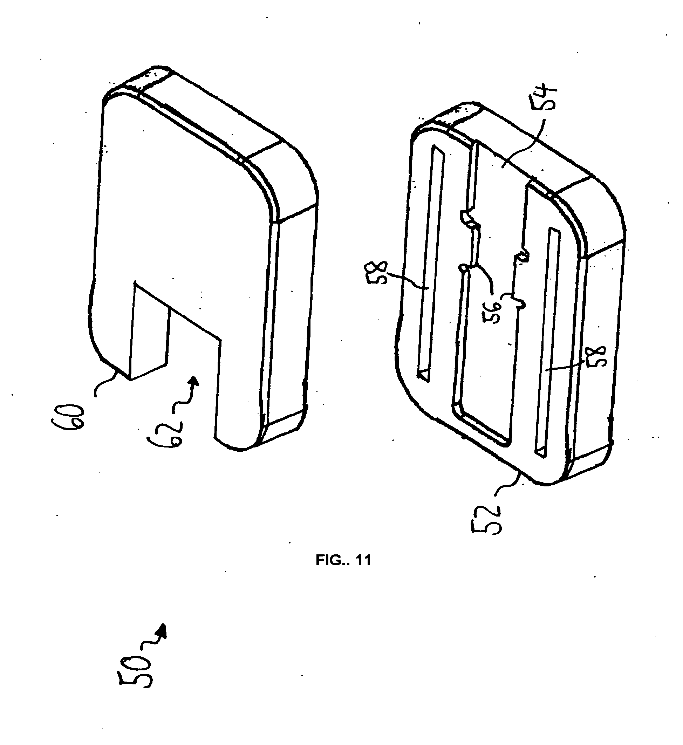

- Fig. 11: zeigt eine perspektivische Ansicht eines nicht erfindungsgemäßen siebten Separators mit einem Unterteil und einem von diesem abgenommenen Oberteil gemäss einem Ausführungsbeispiel;

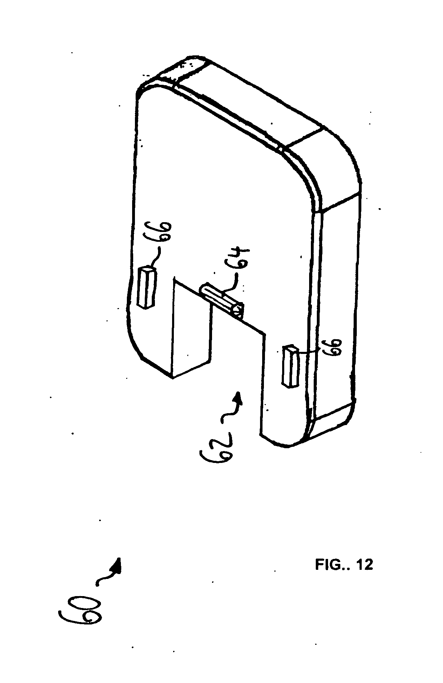

- Fig. 12: zeigt eine perspektivische Ansicht des Oberteils aus Fig. 11 schräg von unten; und

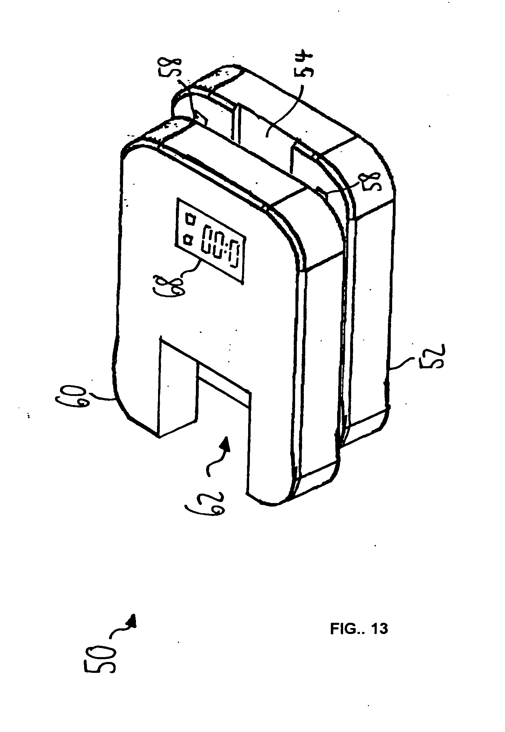

- Fig. 13: zeigt eine perspektivische Ansicht des siebten Separators mit dem auf das Unterteil aufgesetzten Oberteil in einer maximalen Verschiebeposition.

Fig. 1a zeigt eine perspektivische Ansicht eines ersten erfindungsgemäßen Separators 1a in einer ersten Ausführungsform im aufgeklappten Zustand ohne einen eingelegten Separationsstreifen.

Der erste Separator 1a umfasst ein Unterteil 4 und ein Oberteil 3, das in einer nahezu senkrechten Position zu dem Unterteil 4 dargestellt ist. In seinem unteren Bereich weist das Oberteil 3 rechtwinklig an dem Oberteil 3 ansetzende Gelenkvorsprünge auf. Durch diese Gelenkvorsprünge und durch den von diesen Gelenkvorsprüngen eingeschlossenen, in Fig. 1a links angeordneten stufenförmigen End - abschnitt des Unterteils 4 verlaufen Bohrungen, durch die ein Gelenkstift 15 geführt ist, wodurch das Gelenk 5 gebildet wird, um das das Unterteil 4 und das Oberteil 3 geschwenkt und geklappt werden können.

Die Materialdicke und die Abmessungen des Oberteils 3 und des Unterteils 4 stimmen in etwa überein. Die sich in Fig. 1a nach rechts erstreckenden Kanten des Unterteils 4 sind abgerundet, ebenso verhält es sich mit den in Fig. 1a in oberer Richtung dargestellten Kanten des Oberteils 3. Dementsprechend fluchten die Seitenflächen des Oberteils 3 und des Unterteils 4 im zusammengeklappten Zustand in etwa miteinander.

Wie in Fig. 1a gut zu erkennen ist, weist das Oberteil 3 eine Aussparung 8 nahe dem Gelenk auf, so dass im zusammengeklappten Zustand des ersten Separators 1a dass das hintere Ende der Oberseite des Unterteils 4 von oben zugänglich ist.

Auf der Oberseite des Unterteils 4 ist eine Vertiefung 6 ausgebildet, die sich von vorne nach hinten über die Oberseite des Unterteils 4 erstreckt und nach hinten, nach links und nach rechts durch Seitenwände begrenzt und nach vorne hin offen ist. Die in Fig. 1a hinten dargestellte Seitenwand kann auch einen Durchbruch aufweisen, damit das separierte Blutserum oder Blutplasma leichter entnommen werden kann.

An der linken und der rechten Seitenwand der Vertiefung 6 sind einander gegenüberliegende, nasenförmige Ausformungen 9 ausgebildet sind, die in die Vertiefung 6 seitlich hinein ragen und somit deren Breite in diesem Bereich verringern. Die beiden nasenförmigen Ausformungen 9 sind in Fig. 1a in einem vorderen Bereich der Vertiefung 6 angeordnet, und sie stellen Anschläge 9 für einen erfindungsgemäßen Separationsstreifen dar, der nachfolgend noch näher erläutert wird.

An dem Oberteil 3, direkt benachbart der rechteckigen Aussparung 8 ist ein schwellenförmig ausgebildeter und quer zu der Vertiefung 6 verlaufender, erster Vorsprung 7a ausgebildet, der in Fig. 1a nach vorne weist und im zusammengeklappten Zustand des ersten Separators 1a demzufolge nach unten gerichtet ist.

Auf dem Boden der Vertiefung 6 ist in einem hinteren Bereich der Vertiefung 6 ein zweiter Vorsprung 7b angeordnet, der in Fig. 1a nach oben aus der Vertiefung 6 herausragt. Dieser zweite Vorsprung 7b ist so angeordnet, dass er im zusammengeklappten Zustand des ersten Separators 1a mit dem ersten Vorsprung 7a zusammenwirkt. Der zweite Vorsprung 7b ist ebenfalls schwellenförmig und quer zur Längsrichtung der Vertiefung 6 angeordnet.

Die Vorsprünge 7a und 7b in Fig. 1a sind nur beispielhaft, sie können beliebig ausgebildet sein, bspw. kann auch einer der beiden Vorsprünge eine grössere Breite haben oder die Vorsprünge können eine gekrümmte oder gerundete Form aufweisen.

In dem Oberteil 3 ist eine Zeitmesseinheit 48 integriert, die in Fig. 1a eine Digitalanzeige und zwei rechts davon angeordnete Bedientasten umfasst. Mittels der Zeitmesseinheit 48 kann ein Zeitintervall gestoppt werden, das erforderlich ist, damit sich in dem Separationsstreifen das Blutserum oder Blutplasma durch Kapillarwirkung von den roten Blutkörperchen trennt und an dem vorderen Ende des Blutseparationsteils nahe der Blutentnahmeöffnung ansammelt. Nach Ablauf dieses Zeitintervalls kann durch den Separator 1a das Blutserum oder Blutplasma aus dem Separationsstreifen herausgestreift werden.

Mittels den Bedientasten kann das Zeitintervall manuell eingestellt werden. Beispielsweise ist für eine Blutprobe eines Erwachsenen ein Zeitintervall von etwa 0,5 Minuten, für eine Blutprobe eines Neugeborenen ein Zeitintervall von etwa 1 Minute und für eine Blutprobe eines Frühgeborenen ein Zeitintervall von etwa 2 Minuten erforderlich.

Der Zeitintervall beginnt mit Einführen einer Blutprobe in den Separationsstreifen. Der Beginn der Zeitintervallmessung wird der Zeitmesseinheit 48 durch Betätigen einer Bedientaste der Zeitmesseinheit 48 angezeigt. Alternativ dazu kann der Beginn der Zeitintervallmessung auch ab dem Zeitpunkt des Zusammenklappens von Oberteil 3 und Unterteil 4 erfolgen, was durch einen in der Figur 1a nicht gezeigten Schalter oder Sensor erfasst wird.

Nach Ablauf des Zeitintervalls kann durch die Zeitmesseinheit 48 ein akustisches oder optisches Signal ausgegeben werden, das signalisiert, dass nun der Separationsstreifen zurückgezogen werden kann, um das Blutserum oder Blutplasma herauszustreifen.

In der einfachsten Ausführungsform der Zeitmesseinheit 48 kann diese anstelle einer Digitalanzeige über eine LED, die nach Ablauf des Zeitintervalls zu leuchten oder zu blinken beginnt, und/oder über einen Summer verfügen, der nach Ablauf des Zeitintervalls ein Summton erzeugt.

Obwohl die Zeitmesseinheit 48 nur bei dem ersten Separator 1a gezeigt ist, kann die Zeitmesseinheit 48 auch bei den folgenden Separatoren 1b, 1c, 1d und 1e integiert sein. Auf eine separate Beschreibung der Zeitmesseinheit 48 mit Bezug auf die Separatoren 1b, 1c, 1d und 1 e wird verzichtet, um Wiederholungen zu vermeiden.

Fig. 1b zeigt eine perspektivische Ansicht eines zweiten Separators 1 b gemäss einem zweiten Ausführungsbeispiel im aufgeklappten Zustand ohne einen eingelegten Separationsstreifen.

Der zweite Separator 1b unterscheidet sich vom ersten Separator 1a dadurch, dass anstelle des ersten Vorsprungs 7a und des zweiten Vorsprungs 7b ein halbkreisförmig ausgebildeter dritter Vorsprung 27a und ein halbkreisförmig ausgebildeter vierter Vorsprungs 27b vorgesehen sind. Die Öffnungen des Halbkreises des dritten Vorsprungs 27a und des vierten Vorsprungs 27b zeigen in die Richtung des Gelenks 5.

Fig. 1c zeigt eine perspektivische Ansicht eines dritten Separators 1 c gemäss einem dritten Ausführungsbeispiel im aufgeklappten Zustand ohne einen eingelegten Separationsstreifen.

Der dritte Separator 1c unterscheidet sich vom ersten Separator 1a dadurch, dass anstelle des ersten Vorsprungs 7a und des zweiten Vorsprungs 7b ein V-förmiger fünfter Vorsprung 37a und ein V-förmiger sechster Vorsprung 37b vorgesehen sind, welche die Form von Schenkeln eines gleichschenkligen Dreiecks haben, wobei die Spitze des Dreiecks, an der die beiden Schenkel miteinander verbunden sind, von dem Gelenk 5 weg zeigt.

Fig. 1d zeigt eine perspektivische Ansicht eines vierten Separators 1d gemäss einem vierten Ausführungsbeispiel im aufgeklappten Zustand ohne einen eingelegten Separationsstreifen.

Der vierte Separator 1d unterscheidet sich von dem ersten Separator 1a, dass anstelle des ersten Vorsprungs 7a und des zweiten Vorsprungs 7b eine erste Rolle bzw. Walze 47a und eine zweite Rolle bzw. Walze 47b vorgesehen sind. Durch das Vorsehen der Rollen 47a und 47b kann der Separationsstreifen mit weniger Kraftaufwand und daher leichter bewegt und verschoben werden.

Die erste Rolle 47a und die zweite Rolle 47b sind um eine ungefähr auf Höhe der Unterseite des Oberteils 3 und der Oberseite des Unterteils 4 angeordnete und insbesondere in dem Oberteil 3 und dem Unterteil 4 angeordnete, rechtwinklig zur Längsrichtung der Vertiefung 6 bzw. zur Bewegungsrichtung des Separationsstreifens ausgerichtete erste Achse 7c sowie zweite Achse 7d drehbar gelagert, sodass sie bei einer Bewegung oder Verschiebung des Separationsstreifens in eine Drehbewegung versetzt werden.

Alternativ dazu kann wenigstens ein in Figur 1d nicht gezeigter elektrischer Antriebsmotor vorgesehen sein, der im Betrieb die Rollen 47a und 47b antreibt. Dieser Antriebsmotor ist dabei vorteilhafterweise in das Oberteil 3 oder in das Unterteil 4 des vierten Separators 1d integriert. Wenn beide Rollen 47a und 47b angetrieben werden sollen, kann ein gemeinsamer Antriebsmotor in dem Oberteil 3 oder in dem Unterteil 4 vorgesehen werden, oder es kann je ein elektrischer Antriebsmotor für die Rolle 47a in dem Oberteil 3 und für die Rolle 47b in dem Unterteil 4 vorgesehen werden.

Fig. 1e zeigt eine perspektivische Ansicht eines fünften Separators 1 e gemäss einem fünften Ausführungsbeispiel im aufgeklappten Zustand ohne einen eingelegten Separationsstreifen.

Der fünfte Separator 1e unterscheidet sich von dem ersten Separator 1a dadurch, dass Permanentmagnete 22 in den abgerundeten Ecken des Oberteils 3 sowie in den abgerundeten Ecken des Unterteils 4 angeordnet sind. Diese haben in der beispielhaften Darstellung gemäss Fig. 1e einen kreisförmigen Querschnitt uns sind derart angeordnet, dass sie im zusammengeklappten Zustand des fünften Separators 1e einander gegenüber liegen. Die Permanentmagnete 22 des Oberteils 3 und die Permanentmagnete 22 des Unterteils 4 sind jeweils entgegengesetzt magnetisiert, sodass sie sich gegenseitig anziehen. Dadurch wirkt beim Zusammenklappen des fünften Separators 1e eine definierte Kraft zwischen Oberteil 3 und Unterteil 4, welche ein manuelles Zusammendrücken von Oberteil 3 und Unterteil 4 ersetzt.

Fig. 1f zeigt eine perspektivische Ansicht eines sechsten Separators 1f gemäss einem sechsten Ausführungsbeispiel im aufgeklappten Zustand ohne einen eingelegten Separationsstreifen.

Der sechste Separator 1f unterscheidet sich vom dritten Separator 1 c dadurch, dass die V-förmigen Vorsprünge 38a und 38b die Form von Schenkeln eines gleichschenkligen Dreiecks haben, wobei die Spitze des Dreiecks, an der die beiden Schenkel miteinander verbunden sind, zu dem Gelenk 5 hin zeigt.

Fig. 2 zeigt einen ersten, nicht erfindungsgemäßen, Separationsstreifen 2a gemäss einem ersten Ausführungsbeispiel in einer Schnittdarstellung (Fig. 2a) und in einer Draufsicht (Fig. 2b).

Dieser erste Separationsstreifen 2a ist zum Abscheiden von Blutserum oder Blutplasma aus einer Blutprobe bestimmt. Im mittleren Bereich der Fig. 2a ist ein Blutseparationsteil 11 dargestellt, der von einem Halteteil 10 umgeben wird. Das Halteteil 10 umfasst ein unteres Halteteil 10a und ein oberes Halteteil 10b, die das Blutseparationsteil 11 umschliessen. In dem in Fig. 2a links dargestellten vorderen Abschnitt des oberen Halteteils 10b ist ein erster Bluteinführungsabschnitt 12a ausgebildet, durch den eine Blutprobe in das Blutseparationsteil 11 eingeführt werden kann. In dem in Fig. 2a rechts dargestellten hinteren Abschnitt des oberen Halteteils 10b ist eine Blutentnahmeöffnung 13 ausgebildet.

Fig. 2b zeigt das Halteteil 10, welches das in der Draufsicht gestrichelt dargestellte Blutseparationsteil 11 umgibt. Der erste Bluteinführungsabschnitt 12a ist in dem in Fig. 2b links dargestellten vorderen Endabschnitt des Blutseparationsteils 11 gut zu erkennen.

Fig. 3 zeigt eine Draufsicht auf einen zweiten, erfindungsgemäßen, Separationsstreifen 20a.

Der zweite Separationsstreifen 20a unterscheidet sich von dem ersten Separationsstreifen 2a, wie er in den Fig. 2a und 2b dargestellt ist, dadurch, dass an den Längsseiten des zweiten Separationsstreifens 20a jeweils rechteckige Verengungen 14 mit einem hinteren Anschlag 14a und mit einem vorderen Anschlag 14b ausgebildet sind. Diese Anschläge 14a und 14b schlagen in dem in den Separator eingelegten Zustand jeweils an die nasenförmigen Ausformungen 9 an, so dass der zweite Separationsstreifen 20a zwischen zwei definierten Positionen bzgl. des Separators bewegt werden kann.

Der in den Fig. 2 und Fig. 3 als erster Bluteinführungsabschnitt 12a bezeichnete Bereich des Separationsstreifens 2a bzw. 20a kann auch als Separationsfeld bezeichnet werden. Die Separationsstreifen 2a und 20a sind auf ihrer Unterseite mit einer in den Fig. 2 und Fig. 3 nicht zu erkennenden laufenden Nummer versehen.

Fig. 4 zeigt eine Draufsicht auf einen dritten, nicht erfindungsgemäßen, Separationsstreifen 2b, und Fig. 5 zeigt eine Draufsicht auf einen vierten erfindungsgemäßen, nicht Separationsstreifen 20b.

Der dritte Separationsstreifen 2b und der vierte Separationsstreifen 20b unterscheiden sich vom ersten Separationsstreifen 2a und vom zweiten Separationsstreifen 20a nur durch deren Bluteinführungsabschnitt 12b, dessen hintere Seite halbkreisförmig ausgebildet ist.

Fig. 6 zeigt eine Draufsicht auf einen fünften, nicht erfindungsgemäßen, Separationsstreifen 2c, und Fig. 7 zeigt eine Draufsicht auf einen sechsten erfindungsgemäßen Separationsstreifen 20c.

Der fünfte Separationsstreifen 2c und der sechste Separationsstreifen 20c unterscheiden sich vom ersten Separationsstreifen 2a und vom zweiten Separationsstreifen 20a nur durch deren Bluteinführungsabschnitt 12c, dessen hintere Seite V-förmig ausgebildet ist.

Fig. 8 zeigt eine perspektivische Ansicht des ersten Separators 1a im aufgeklappten Zustand mit eingelegtem zweiten Separationsstreifen 20a.

Dabei ist an der Oberseite des zweiten, erfindungsgemäßen, Separationsstreifens 20a der erste Bluteinführungsabschnitt 12a gut zu erkennen. Die ebenfalls an der Oberseite des zweiten Separationsstreifens 20a angeordnete Blutentnahmeöffnung 13 ist an dem in Fig. 8 links dargestellten hinteren Ende des zweiten Separationsstreifens 20a gestrichelt dargestellt. Der in der Vertiefung 6 ausgebildete zweite Vorsprung 7b ist in Fig. 4 demgemäss nicht zu erkennen, zumal er von dem zweiten Separationsstreifen 20a verdeckt wird.

In Fig. 8 ist ebenfalls gut zu erkennen, dass die vorderen Anschläge 14b der Verengung 14 des zweiten Separationsstreifens 20a an den nasenförmigen Ausformungen 9 anliegen, und dass sich der zweite Separationsstreifen 20a somit in der eingeschobenen Position befindet.

Fig. 9a zeigt eine Darstellung eines Längsschnitts des ersten Separators 1a im aufgeklappten Zustand.

Im oberen Teil ist das senkrecht angeordnete Oberteil 3 zu erkennen, das durch den Gelenkstift 15 schwenkbar mit dem in Fig. 9a waagerecht ausgerichteten Unterteil 4 verbunden ist. An der nach hinten weisenden Oberseite des Oberteils 3 ist eine Ausnehmung 16 angeordnet, in die bspw. eine Typenbezeichnung oder ein Firmenlogo eingebracht ist. An der Unterseite des Oberteils 3 ist der erste Vorsprung 7a zu erkennen, der nach vorne weist. An der Oberseite des Unterteils 4 sind die an den Seitenwänden der Vertiefung ausgebildeten nasenförmigen Ausformung 9 ebenso zu erkennen, wie der am Boden der Vertiefung 6 ausgebildete zweite Vorsprung 7b.

Fig. 9b zeigt eine Seitenansicht des ersten Separators 1a im aufgeklappten Zustand.

Dabei sind das waagrecht angeordnete Unterteil 4 und das dazu senkrecht angeordnete Oberteil 3 zu erkennen. Des weiteren ist der an der Unterseite des Oberteils 3 angeordnete erste Vorsprung 7a zu erkennen.

Fig. 10 zeigt eine perspektivische Ansicht des Gelenkstifts 15.

Dabei umfasst der Gelenkstift 15 einen Kopf 18 mit einer Stirnfläche 19, in die ein Schlitz 20 eingebracht ist, in den ein Schraubenzieher eingreifen und so den Gelenkstift 15 bewegen oder drehen kann. Des weiteren verfügt der Gelenkstift 15 über einen Bolzen 17 der einen geringeren Durchmesser aufweist als der Kopf 18 und der von dem Kopf 18 durch eine Einkerbung 21 beabstandet ist. In Fig. 10 ist der Bolzen 17 verkürzt dargestellt. In der Praxis ist der Bolzen so lang ausgebildet, dass er sich von dem Kopf 18 durch das Unterteil 4 bis zu dem gegenüberliegenden Gelenkabschnitt des Oberteils 3 erstreckt.

Der Separator 1a, 1b, 1c, 1d, 1e und 1f mit dem zweiten Separationsstreifen 20a wird wie folgt bedient:
Zunächst wird eine Blutprobe von etwa 50 µl Blut bspw. mit einer Pipette auf den ersten Bluteinführungsabschnitt 12a gegeben. Dabei wird durch Kapillarwirkung das Blutserum oder Blutplasma von den roten Blutkörperchen so getrennt, dass sich das Blutserum oder Blutplasma an dem hinteren Ende des Blutseparationsteils 11 nahe der Blutentnahmeöffnung 13 ansammelt.
Danach wird der zweite Separationsstreifen 20a, wie in Fig. 8 dargestellt, mit nach oben weisendem ersten Bluteinführungsabschnitt 12a so in den Separator 1a, 1b, 1c, 1d, 1e und 1f gelegt, dass die vorderen Anschläge 14b an den nasenförmigen Ausformungen 9 anschlagen. Der zweite Separationsstreifen 20a befindet sich somit im eingeschobenen Zustand.

Nach Ablauf eines Zeitintervalls von etwa 0,5 bis 2 Minuten, je nach Analyt, werden das Oberteil 3 und das Unterteil 4 zusammengeklappt, und der Separator wird somit geschlossen. Das Oberteil 3 und das Unterteil 4 werden leicht aufeinandergedrückt gehalten und gleichzeitig wird der zweite Separationsstreifen 20a vorsichtig zurückgezogen, bis die hinteren Anschläge 14a an die nasenförmigen Ausformungen 9 anschlagen. Bei Verwendung des fünften Separators 1e mit Permanentmagneten 22 kann das manuelle Aufeinanderdrücken von Oberteil 3 und Unterteil 4 entfallen, da Oberteil 3 und Unterteil 4 durch magnetische Kraftwirkung gegeneinander gedrückt werden.

Durch die Einwirkung der Vorsprünge 7a und 7b auf das Blutseparationsteil 11 wird das Blutserum oder Blutplasma nun aus der Blutentnahmeöffnung 13 herausgestreift und tritt an der Spitze des zweiten Separationsstreifens 20a aus.

Bei Verwendung des zweiten Separators 1b, des dritten Separators 1 c oder des sechsten Separators 1f wird durch die halbkreisförmigen Vorsprünge 27a und 27b bzw. durch die V-förmigen Vorsprünge 37a und 37b bzw. 38a und 38b beim Herausziehen des zweiten Separationsstreifens 20a aus der Vertiefung 6 das Blutserum oder Blutplasma in der Mitte des zweiten Separationsstreifens 20a konzentriert und mittig aus dem zweiten Separationsstreifen 20a herausgedrückt.

Bei Verwendung des vierten Separators 1d wird durch die Rollen 47a und 47b beim Herausziehen des zweiten Separationsstreifens 20a aus der Vertiefung 6 das Blutserum oder Blutplasma besonders schonend aus dem zweiten Separationsstreifen 20a herausgedrückt.

Wird anstelle des zweiten Separationsstreifens 20a ein vierter Separationsstreifen 20b mit einem halbkreisförmigen Bluteinführungsabschnitt 12b oder ein sechster Separationsstreifen 20c mit einem V-förmigen Bluteinführungsabschnitt 12c verwendet, so wird das Blutserum oder Blutplasma noch besser in der Mitte des Bluteinführungsabschnitt 12b und 12c konzentriert und herausgedrückt.

Das Blutserum oder Blutplasma kann nun berührungsfrei entnommen werden, bspw. kann es mit einer Kapillarküvette aufgesaugt werden. Schliesslich ist die Kapillarküvette insbesondere mit einem Verschlusskit zu versiegeln, um das Ausfliessen des Blutserums oder des Blutplasmas zu verhindern. Die Kapillarküvette ist nun zur Messung bereit.

Fig. 11 zeigt eine perspektivische Ansicht eines siebten, nicht erfindungs gemäßen, Separators 50 mit einem Unterteil 52 und einem von diesem abgenommenen Oberteil 60.

Das Unterteil 52 und das Oberteil 60 haben jeweils die gleiche Dicke und eine jeweils im Wesentlichen rechteckige Form mit abgerundeten Ecken, wobei im mittleren Bereich des hinteren Endes des Oberteils 60 eine rechteckige Aussparung 62 angeordnet ist. In den Seitenbereichen der Oberfläche des Unterteils 52 sind zwei längliche Führungsschienen 58 ausgebildet, die in Fig. 11 in Form von Längsnuten ausgebildet sind. Auf der Oberseite des Unterteils 52 ist eine längliche Vertiefung 54 ausgebildet, die sich von vorne nach hinten über die Oberseite des Unterteils 52 erstreckt und die nach hinten, nach links und nach rechts durch Seitenwände begrenzt und nach vorne hin offen ist. Die hintere Seitenwand kann dabei auch einen Durchbruch aufweisen, damit das separierte Blutserum oder Blutplasma leichter entnommen werden kann.

An der linken und der rechten Seitenwand der Vertiefung 54 sind in einem vorderen Bereich einander gegenüberliegende Fixierungsnasen 56 angeordnet, deren Breite jeweils der Breite der Ausschnitte 14 der Separationsstreifen 20a, 20b und 20c entspricht. Durch diese Fixierungsnasen 56 kann ein in die Vertiefung 54 eingelegter Separationsstreifen 20a, 20b, 20c zuverlässig fixiert und in Position gehalten werden.

Fig. 12 zeigt eine perspektivische Ansicht des Oberteils 60 schräg von unten.

Direkt vor der rechteckigen Aussparung 62 an der Hinterseite des Oberteils 60 ist eine Rolle 64 angeordnet, die um eine ungefähr auf der Höhe der Unterseite des Oberteils 60 angeordnete sowie rechtwinklig zur Längsrichtung der Vertiefung 54 bzw. zur Bewegungsrichtung des Separationsstreifens ausgerichtete Achse drehbar gelagert ist. Die Rolle 64 steht somit ein Stück weit nach unten aus der Unterseite des Oberteils 60 heraus, so dass die Rolle 64 bei einer Verschiebebewegung des Separationsstreifens abrollt und unter Druckeinwirkung auf den Separationsstreifen das Blutserum oder Blutplasma aus diesem herausstreift.

An den Seitenbereichen des Oberteils 60 neben der Aussparung 62 ragen zwei Gleiter 66 nach unten aus dessen Oberfläche heraus. Diese Gleiter 66 können in die Führungsschienen 58 des Unterteils 52 eingesetzt werden und führen das Oberteil 60 während seiner Schiebebewegung in den Führungsschienen 58. Die Gleiter 66 sind in Bezug auf die Längsrichtung des sechsten Separators 50 hinter der Rolle 64 angeordnet, so dass sie an die hinteren Enden der Führungsschienen 58 anschlagen und die Verschiebebewegung des Oberteils 60 nach hinten begrenzen, so dass die Rolle 64 das Blutserum oder Blutplasma aus der Blutentnahmeöffnung 13 zwar herausstreift, aber nicht mit dem herausgestreiften Blutserum oder Blutplasma im hinteren Abschnitt des Separationsstreifens 20a, 20b, 20c in Berührung kommt.

Durch die oberteilseitigen Gleiter 66 und die unterteilseitigen Führungsschienen 58 wird somit ein Verschiebemechanismus gebildet, mit dem das Oberteil 60 gegenüber dem Unterteil 52 verschoben werden kann.

Dieser Verschiebemechanismus ist in den Figuren 11 bis 13 nur beispielhaft angegeben, er kann auf beliebige Weise ausgeführt werden. Beispielsweise können Führungsschienen in den Seitenflächen des Unterteils 52 vorgesehen sein, und das Oberteil 60 kann breiter als das Unterteil 52 ausgebildet sein und Gleiter aufweisen, die in die seitlichen Führungsschienen des Unterteils 52 eingreifen. Dabei ist das Oberteil 60 gegenüber dem Unterteil 52 so weit nach hinten verschiebbar, dass die Vertiefung 54 freiliegt und der Separationsstreifen 20a, 20b, 20c eingelegt werden kann. Wie bei der Ausführung gemäß den Figuren 11 bis 13 können die Gleiter in Bezug auf die Längsrichtung so angeordnet sein, so dass sie an die hinteren Enden der seitlichen Führungsschienen anschlagen und die Verschiebebewegung des Oberteils 60 nach hinten begrenzen, so dass die Rolle 64 das Blutserum oder Blutplasma aus der Blutentnahmeöffnung 13 zwar herausstreift, aber nicht mit dem herausgestreiften Blutserum oder Blutplasma im hinteren Abschnitt des Separationsstreifens 20a, 20b, 20c in Berührung kommt.

Fig. 13 zeigt eine perspektivische Ansicht des siebten, nicht erfindungsgemäßen, Separators 50 mit dem auf das Unterteil 52 aufgesetzten Oberteil 60 in einer maximalen Verschiebeposition.

Dabei sind, was in Fig. 13 nicht zu sehen ist, die Gleiter 66 an die hinteren Enden der Führungsschienen 58 angeschlagen, und das hintere Ende des Oberteils 60 steht über dasjenige des Unterteils 52 heraus.

Des weiteren ist auf der Oberseite des Oberteils 60 in einem vorderen Bereich eine Zeitmesseinheit 68 mit einer Digitalanzeige und mit zwei Bedientasten angeordnet, die der Zeitmesseinheit 48 des ersten Separators 1 a entspricht.

Der siebte, nicht erfindungsgemäßen, Separator 50 mit dem Separationsstreifen 20a, 20b, 20c wird wie folgt bedient:
Zunächst wird der Separationsstreifen 20a, 20b, 20c mit nach oben weisendem ersten Bluteinführungsabschnitt 12a so in den Separator 50 gelegt, dass die Fixierungsnasen 56 in den Ausschnitt 14 eingreifen und dass der Separationsstreifen 20a, 20b, 20c so gegenüber dem Unterteil 52 fixiert und in Position gehalten wird. Dann wird eine Blutprobe von etwa 50 µl Blut beispielsweise mit einer Pipette auf den ersten Bluteinführungsabschnitt 12a, 12b, 12c gegeben, und das Blutserum bzw. das Blutplasma trennt sich durch Kapillarwirkung von den roten Blutkörperchen derart, dass es sich an dem hinteren Ende des Blutseparationsteils 11 nahe der Blutentnahmeöffnung 13 ansammelt. Nun wird das Oberteil 60 fluchtend auf das Unterteil 52 aufgesetzt, so dass die Gleiter 66 in den Führungsschienen 58 angeordnet sind. Entweder durch Tastendruck oder durch einen Schalter oder Sensor, der das Aufsetzen des Oberteils 60 auf das Unterteil 52 erkennt, wird die Zeitmesseinheit 68 gestartet. Nach Ablauf des vorgegebenen Zeitintervalls gibt dieser ein akustisches oder optisches Signal aus. Danach wird das Oberteil 60 manuell oder motorisch über einen integrierten Elektromotor gegenüber dem Unterteil 52 nach hinten verschoben, bis die Gleiter 66 gegen die Enden der Führungsschienen 58 anschlagen. Dabei wird durch das Einwirken der Rolle 64 auf das Blutseparationsteil 11 das Blutserum bzw. das Blutplasma aus der Blutentnahmeöffnung 13 herausgestreift und tritt an der Spitze des Separationsstreifens 20a, 20b, 20c aus.

Das Blutserum oder das Blutplasma kann nun durch die Aussparung 62 des Oberteils 60 entnommen werden, beispielsweise kann es mit einer Kapillarküvette aufgesaugt werden. Schließlich ist die Kapillarküvette insbesondere mit einem Verschlusskit zu versiegeln, um das Ausfließen des Blutserums zu verhindern. Die Kapillarküvette ist nun zur Messung bereit.

## Patentansprüche

1. Separator (1a-f) zur Entnahme von Blutserum oder Blutplasma einer Blutprobe aus einem Separationsstreifen (2a-c; 20a-c),
mit einem Oberteil (3), mit einem Unterteil (4) und mit einem Gelenk (5),
wobei das Oberteil (3) und das Unterteil (4) durch das Gelenk (5) schwenkbar miteinander verbunden sind, so dass der Separator auf- und zuklappbar ist,
wobei in dem zugeklappten Zustand die Unterseite des Oberteils (3) und die Oberseite des Unterteils (4) wenigstens teilweise aneinander anliegen,
wobei in dem Unterteil (4) eine Vertiefung (6) zur Aufnahme des Separationsstreifens (2a-c; 20a-c) ausgebildet ist, die an zwei gegenüberliegenden Seiten durch Seitenwände begrenzt ist, wobei an jeder der gegenüberliegenden Seitenwände jeweils eine nasenförmige Ausformung (9) als Anschlag für den in die Vertiefung (6) eingelegten Separationsstreifen ausgebildet ist, die seitlich in die Vertiefung hineinragt, und
wobei an dem Unterteil (4) und/oder an der Unterseite des Oberteils (3) wenigstens ein Vorsprung (7a-b; 27a-b; 37a-b; 38a-b; 47a-b) zum Ausüben eines definierten, lokalen Drucks auf den Separationsstreifen (2a-c; 20a-c) bei Zusammenklappen von Oberteil (3) und Unterteil (4) ausgebildet ist, so dass beim Verschieben des Separationsstreifens (2a-c; 20a-c) Blutserum oder Blutplasma aus dem hinteren Ende des Separationsstreifens (2a-c; 20a-c) austreten kann.

2. Separator (1a-f) nach Anspruch 1,
**dadurch gekennzeichnet, dass**
der Separationsstreifen (2a-c) in die Vertiefung (6) einschiebbar und aus der Vertiefung (6) herausziehbar ist.

3. Separator (1a-f) nach Anspruch 1 oder 2,
**dadurch gekennzeichnet, dass**
ein bzw. der Vorsprung (7b; 27b; 37b; 38b; 47b) an der Vertiefung (6), insbesondere auf dem Boden der Vertiefung (6) ausgebildet ist.

4. Separator (1a-f) nach einem der Ansprüche 1 bis 3,
**dadurch gekennzeichnet, dass**
an der Unterseite des Oberteils (3) ein erster Vorsprung (7a; 27a; 37a; 38a; 47a) und auf dem Boden der Vertiefung (6) ein zweiter Vorsprung (7b; 27b; 37b; 38b; 47b) ausgebildet sind, die im zusammengeklappten Zustand des Separators (1a-f) zusammenwirken.

5. Separator (1a-f) nach einem der Ansprüche 1 bis 4,
**dadurch gekennzeichnet, dass**
der wenigstens eine Vorsprung (7a-b; 27a-b; 37a-b; 38a-b) schwellenförmig ausgebildet ist.

6. Separator (1d) nach einem der Ansprüche 1 bis 4,
**dadurch gekennzeichnet, dass**
der wenigstens eine Vorsprung (47a-b) als Rolle oder Walze ausgebildet ist, die um eine in das Oberteil (3) bzw. in das Unterteil (4) eingelassene Achse rotierbar ist.

7. Separator (1d) nach Anspruch 6,
**dadurch gekennzeichnet, dass**
ein Antriebsmotor für die Rolle oder Walze vorgesehen ist.

8. Separator (1a-f) nach einem der Ansprüche 1 bis 7,
**dadurch gekennzeichnet, dass**
das Oberteil (3) nahe dem wenigstens einen Vorsprung (7a-b; 27a-b; 37a-b; 38a-b; 47a-b) eine Aussparung (8) hat, so dass das separierte Blutserum oder Blutplasma im zusammengeklappten Zustand des Separators (1a-f) zugänglich ist.

9. Separator (1e) nach einem der Ansprüche 1 bis 8,
**dadurch gekennzeichnet, dass**
Permanentmagnete (22) derart in dem Oberteil (3) sowie in dem Unterteil (4) ausgebildet sind, dass die Permanentmagnete (22) im zusammengeklappten Zustand einander gegenüberliegend angeordnet sind und sich gegenseitig anziehen.

10. Separator (1e) nach einem der Ansprüche 1 bis 8,
**dadurch gekennzeichnet, dass**
wenigstens ein Permanentmagnet (22) in dem Oberteil (3) ausgebildet ist und das Unterteil (4) Metall aufweist.

11. Separator (1e) nach einem der Ansprüche 1 bis 8,
**dadurch gekennzeichnet, dass**
wenigstens ein Permanentmagnet (22) in dem Unterteil (4) ausgebildet ist und das Oberteil (3) Metall aufweist.

12. Separationsstreifen (20a-c) zum Abscheiden von Blutserum oder Blutplasma aus einer Blutprobe mittels eines Separators (1a-f), umfassend:
einen Blutseparationsteil (11),
einen Halteteil (10) zum Abdecken und Festhalten des Blutseparationsteils (11),
einen Bluteinführungsabschnitt (12a-c), der in einem das vordere Ende des Blutseparationsteils (11) abdeckenden Abschnitt des Halteteils (10) geformt ist, und
eine Blutentnahmeöffnung (13), die in dem das hintere Ende des Blutseparationsteils (11) abdeckenden Abschnitt des Halteteils (10) geformt ist, **dadurch gekennzeichnet, dass**
der Separationsstreifen (20a-c) an wenigstens einer Längsseite wenigstens eine Verengung (14) mit einem hinteren und mit einem vorderen Anschlag (14a, 14b) aufweist, eo dass der Separationsstreifen (20a-c) über die durch die Anschläge (14a, 14b) vorgegebene Distanz bewegbar ist.

13. Separationsstreifen (20a-c) nach Anspruch 12,
**dadurch gekennzeichnet, dass**
die Blutprobe durch den Blutein Führungs abschnitt (12a-c) in den Blutseparationsteil (11) einführbar ist und der Blutseparationsteil (11) derart beschaffen ist, dass die eingeführte Blutprobe durch Kapillarwirkung derart trennbar ist, dass sich das Blutserum oder Blutplasma im hinteren Ende des Blutseparationsteils (11) und die roten Blutkörperchen im vorderen Ende des Blutseparationsteils (11) ansammeln.

14. Separationsstreifen (20a-c) nach Anspruch 12 oder 13,
**dadurch gekennzeichnet, dass**
die wenigstens eine Verengung (14) rechteckig ausgebildet ist.

15. Separationsstreifen (20a-c) nach einem der Ansprüche 12 bis 14,
**dadurch gekennzeichnet, dass**
der Separationsstreifen (20a-c) an einer Seite halbkreisförmig oder V-förmig ist.

## Claims

1. Separator (1a-f) for the taking of blood serum or blood plasma contained in a blood sample from a separating strip (2a-c; 20a-c),
comprising a top part (3), a bottom part (4) and a joint (5),
wherein the top part (3) and the bottom part (4) are linked together by the joint (5) in a pivoting way so that the separator can be folded open and closed by pivoting,
wherein the underside of the top part (3) and top side of the bottom part (4) come into contact with each other at least in some areas in said folded-closed condition,
wherein the bottom part (4) has an indentation (6) for receiving the separating strip (2a-c; 20a-c) which is limited by side walls on at least two opposite sides and wherein at each of the opposite side walls at least one nose-shaped lug (9) is formed as a limit stop laterally protruding into the indentation for the separating strip inserted in the indentation (6), and
wherein at the bottom part (4) and/or at the underside of the top part (3) at least one nose (7a-b; 27a-b; 37a-b; 38a-b; 47a-b) is formed in order to exert a defined local pressure on the separating strip (2a-c; 20a-c) once the top part (3) and bottom part (4) are folded together, such that blood serum or blood plasma can come out of the rear end of the separating strip (2a-c; 20a-c) when the separating strip (2a-c; 20a-c) is moved.

2. Separator (1a-f) according to Claim 1,
**characterized in that**
the separating strip (2a-c) can be pushed into the indentation (6) and pulled out of the indentation (6).

3. Separator (1a-f) according to Claim 1 or 2,
**characterized in that**
a nose/the nose (7b; 27b; 37b; 38b; 47b) is provided at the indentation (6), in particular at the base of the indentation (6).

4. Separator (1a-f) according to any of Claims 1 to 3,
**characterized in that**
a first nose (7a; 27a; 37a; 38a; 47a) is provided on the underside of top part (3) and a second nose (7b; 27b; 37b; 38b; 47b) is provided on the base of the indentation (6), which both interact when the separator (1a-f) is folded closed.

5. Separator (1a-f) according to any of Claims 1 to 4,
**characterized in that**
the at least one nose (7a-b; 27a-b; 37a-b; 38a-b) is shaped like a barrier.

6. Separator (1d) according to any of Claims 1 to 4,
**characterized in that**
the at least one nose (47a-b) is shaped like a roller or roll which rotatable around an axle inserted in the top part (3) and/or the bottom part (4).

7. Separator (1d) according to Claim 6,
**characterized in that**
a drive motor is provided for the roller or roll.

8. Separator (1a-f) according to any of Claims 1 to 7,
**characterized in that**
the top part (3) has an opening (8) close to the at least one nose (7a-b; 27a-b; 37a-b; 38a-b; 47a-b) allowing access to the separated blood serum or blood plasma while the separator (1a-f) is folded closed.

9. Separator (1e) according to any of Claims 1 to 8,
permanent magnets (22) are arranged both in the top part (3) and the bottom part (4) so that the permanent magnets (22) face each other in the folded-down condition and attract each other.

10. Separator (1e) according to any of Claims 1 to 8,
**characterized in that**
at least one permanent magnet (22) is provided in the top part (3) and the bottom part (4) comprises metal.

11. Separator (1e) according to any of Claims 1 to 8,
**characterized in that**
at least one permanent magnet (22) is provided in the bottom part (4) and the top part (3) comprises metal.

12. Separating strip (20a-c) for the separation of blood serum or blood plasma of a blood sample by means of a separator (1a-f), comprising:
a blood separating element (11),
a retaining element (10) to cover and retain the blood separating element (11),
a blood introducing section (12a-c) provided in a section of the retaining element (10) which covers the front end of the blood separating element (11), and
a blood taking opening (13) provided in a section of the retaining element (10) which covers the rear end of the blood separating element (11),
**characterized in that**
the separating strip (20a-c) has at least one narrow (14) on at least one longitudinal side including a rear and a front limit stop (14a, 14b) so that the separating strip (20a-c) is movable across the distance defined by the limit stops (14a, 14b).

13. Separating strip (20a-c) according to Claim 12,
**characterized in that**
the blood sample can be introduced into the blood separating element (11) through the blood inserting section (12a-c) and the blood separating element (11) is configured such that the introduced blood sample can be separated by capillary action so that the blood serum or blood plasma collect in the rear end of the blood separating element (11) and the erythrocytes collect in the front end of the blood separating element (11).

14. Separating strip (20a-c) according to Claim 12 or 13,
**characterized in that**
at least one narrow (14) has a rectangular shape.

15. Separating strip (20a-c) according to any of Claims 12 to 14,
**characterized in that**
the separating strip (20a-c) is shaped like a semicircle or a V on one side.

## Revendications

1. Séparateur (1a à f) pour prélever du sérum sanguin ou du plasma sanguin d'un échantillon de sang à partir d'une bande de séparation (2a à c ; 20a à c),
comprenant une partie supérieure (3), une partie inférieure (4) et une articulation (5),
dans lequel la partie supérieure (3) et la partie inférieure (4) sont raccordées l'une à l'autre à pivotement par l'articulation (5) de manière à pouvoir ouvrir et rabattre le séparateur,
dans lequel, à l'état rabattu, la face inférieure de la partie supérieure (3) et la face supérieure de la partie inférieure (4) s'appliquent l'une sur l'autre au moins en partie,
dans lequel la partie inférieure (4) présente un creux (6) destiné à recevoir la bande de séparation (2a à c ; 20a à c), qui est délimité sur deux faces opposées par des parois latérales et dans lequel il est formé, respectivement sur chacune des parois latérales opposées, un façonnage (9) en forme de nez s'enfonçant latéralement dans le creux sous la forme d'une butée pour la bande de séparation insérée dans le creux (6), et
dans lequel il est formé, sur la partie inférieure (4) et/ou sur la face inférieure de la partie supérieure (3), au moins une saillie (7a à b ; 27a à b ; 37a à b ; 38a à b ; 47a à b) pour exercer une pression locale définie sur la bande de séparation (2a à c ; 20a à c), lorsque la partie supérieure (3) et la partie inférieure (4) sont rabattues, de sorte que du sérum sanguin ou du plasma sanguin puisse sortir de l'extrémité arrière de la bande de séparation (2a à c ; 20a à c) lors du déplacement de la bande de séparation (2a à c ; 20a à c).

2. Séparateur (1a à f) selon la revendication 1,
**caractérisé en ce que**
la bande de séparation (2a à c) peut être insérée dans le creux (6) et en être extraite.

3. Séparateur (1a à f) selon la revendication 1 ou 2,
**caractérisé en ce que**
une ou la saillie (7b ; 27b ; 37b ; 38b ; 47b) est formée dans le creux (6), en particulier dans le fond du creux (6).

4. Séparateur (1a à f) selon l'une quelconque des revendications 1 à 3,
**caractérisé en ce que**
une première saillie (7a ; 27a ; 37a ; 38a ; 47a) est formée sur la face inférieure de la partie supérieure (3) et une seconde saillie (7b ; 27b ; 37b ; 38b ; 47b) au fond du creux (6), qu
i coopèrent ensemble à l'état rabattu du séparateur (1a à f).

5. Séparateur (1a à f) selon l'une quelconque des revendications 1 à 4,
**caractérisé en ce que**
la au moins une saillie (7a à b ; 27a à b ; 37a à b ; 38a à b) se présente sous la forme d'un seuil.

6. Séparateur (1d) selon l'une quelconque des revendications 1 à 4,
**caractérisé en ce que**
la au moins une saillie (47a à b) se présente sous la forme d'un galet ou d'un rouleau, qui peut tourner autour d'un axe encastré dans la partie supérieure (3) ou la partie inférieure (4).

7. Séparateur (1d) selon la revendication 6,
**caractérisé en ce que**
l'on prévoit un moteur d'entraînement pour le galet ou le rouleau.

8. Séparateur (1a à f) selon l'une quelconque des revendications 1 à 7,
**caractérisé en ce que**
la partie supérieure (3) présente un évidement (8) à proximité de la au moins une saillie (7a à b ; 27a à b ; 37a à b ; 38a à b ; 47a à b) de sorte que le sérum sanguin ou le plasma sanguin séparé soit accessible à l'état rabattu du séparateur (1a à f).

9. Séparateur (1e) selon l'une quelconque des revendications 1 à 8,
**caractérisé en ce que**
des aimants permanents (22) sont formés dans la partie supérieure (3) et dans la partie inférieure (4) de sorte que les aimants permanents (22) soient opposés l'un à l'autre à l'état rabattu et s'attirent mutuellement.

10. Séparateur (1e) selon l'une quelconque des revendications 1 à 8,
**caractérisé en ce que**
au moins un aimant permanent (22) est formé dans la partie supérieure (3) et la partie inférieure (4) comporte du métal.

11. Séparateur (1e) selon l'une quelconque des revendications 1 à 8,
**caractérisé en ce que**
au moins un aimant permanent (22) est formé dans la partie inférieure (4) et la partie supérieure (3) comporte du métal.

12. Bande de séparation (20a à c) destinée à séparer le sérum sanguin ou le plasma sanguin d'un échantillon de sang au moyen d'un séparateur (1a à f), comprenant :
une partie séparatrice de sang (11),
une partie de maintien (10) pour recouvrir et retenir la partie séparatrice de sang (11),
une section d'introduction de sang (12a à c), qui est formée dans une section de la partie de maintien (10) recouvrant l'extrémité avant de la partie séparatrice de sang (11), et
un orifice de prélèvement de sang (13), qui est formé dans la section de la partie de maintien (10) recouvrant l'extrémité arrière de la partie séparatrice de sang (11),
**caractérisée en ce que**
la bande de séparation (20a à c) présente, sur au moins une face longitudinale, au moins un rétrécissement (14) avec une butée arrière et une butée avant (14a, 14b) de sorte que la bande de séparation (20a à c) puisse être déplacée sur la distance prédéfinie par les butées (14a, 14b).

13. Bande de séparation (20a à c) selon la revendication 12,
**caractérisée en ce que**
l'échantillon de sang peut être introduit, via la section d'introduction de sang (12a à c), dans la partie séparatrice de sang (11) et la partie séparatrice de sang (11) est structurée de sorte que l'échantillon de sang introduit puisse être séparé par effet capillaire de manière à accumuler le sérum ou le plasma sanguin à l'extrémité arrière de la partie séparatrice de sang (11) et les globules rouges à l'extrémité avant de la partie séparatrice de sang (11).

14. Bande de séparation (20a à c) selon la revendication 12 ou 13,
**caractérisée en ce que**
le au moins un rétrécissement (14) se présente sous la forme d'un rectangle.

15. Bande de séparation (20a à c) selon l'une quelconque des revendications 12 à 14,
**caractérisée en ce que**
la bande de séparation (20a à c) est en forme de demi-cercle ou de V sur une face.
